(19) Europäisches Patentamt
European Patent Office
Office européen des brevets

(11) **EP 3 153 094 B1**

(12) **EUROPEAN PATENT SPECIFICATION**

(45) Date of publication and mention of the grant of the patent:
**26.01.2022  Bulletin 2022/04**

(21) Application number: **16191387.6**

(22) Date of filing: **29.09.2016**

(51) International Patent Classification (IPC):
*A61B 5/00* $^{(2006.01)}$    *A61B 5/021* $^{(2006.01)}$
*A61B 5/024* $^{(2006.01)}$    *A61B 5/1172* $^{(2016.01)}$
*A61B 5/01* $^{(2006.01)}$

(52) Cooperative Patent Classification (CPC):
**A61B 5/02125; A61B 5/02427; A61B 5/1172;**
**A61B 5/6826; A61B 5/6843;** A61B 5/01

(54) **BLOOD PRESSURE MEASURING APPARATUS, AND BLOOD PRESSURE MEASURING APPARATUS USING LIGHT SOURCE SELECTION PROCESS**

BLUTDRUCKMESSVORRICHTUNG UND BLUTDRUCKMESSVORRICHTUNG MIT LICHTQUELLENAUSWAHLVERFAHREN

APPAREIL DE MESURE DE PRESSION ARTERIELLE ET APPAREIL DE MESURE DE PRESSION ARTERIELLE UTILISANT UN PROCESSUS DE SELECTION DE SOURCE LUMINEUSE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **02.10.2015  KR 20150139389**

(43) Date of publication of application:
**12.04.2017  Bulletin 2017/15**

(60) Divisional application:
**21212750.0**

(73) Proprietor: **Samsung Electronics Co., Ltd.**
**Gyeonggi-do 16677 (KR)**

(72) Inventors:
• **KWON, Yong Joo**
  **Gyeonggi-do (KR)**
• **KANG, Jae Min**
  **Seoul (KR)**
• **KIM, Sun Kwon**
  **Gyeonggi-do (KR)**
• **KIM, Youn Ho**
  **Gyeonggi-do (KR)**
• **PARK, Sang Yun**
  **Gyeonggi-do (KR)**

(74) Representative: **Grünecker Patent- und Rechtsanwälte**
**PartG mbB**
**Leopoldstraße 4**
**80802 München (DE)**

(56) References cited:
**CN-A- 101 828 908      US-A1- 2005 228 296**
**US-A1- 2005 261 593      US-A1- 2009 043 180**
**US-A1- 2015 057 511**

• **JING LIU ET AL: "Effects of cuff inflation and deflation on pulse transit time measured from ECG and multi-wavelength PPG", 2015 37TH ANNUAL INTERNATIONAL CONFERENCE OF THE IEEE ENGINEERING IN MEDICINE AND BIOLOGY SOCIETY (EMBC). PROCEEDINGS IEEE PISCATAWAY, NJ, USA, 29 August 2015 (2015-08-29), pages 5973-5976, XP002767819, ISBN: 978-1-4244-9270-1**

## Description

BACKGROUND

1. Field

Apparatuses and methods consistent with exemplary embodiments relate to a blood pressure measuring technology using light sources.

2. Description of the Related Art

A cuff-less blood pressure measuring apparatus measures blood pressure without pressurization. As general blood pressure estimating methods, a method of using a pulse wave velocity and a method of analyzing the shape of pulse waves are provded. US 2015/0057511 A1 and US 2005/0228296 A1 describe optical sensors for health monitoring.

In the method of using a pulse wave velocity, the pulse wave velocity is measured by using a phase difference between an Electrocardiogram (ECG) signal and a Photo-Plethysmography (PPG) signal. In order to measure the ECG signal and the PPG signal, an electrode for the ECG signal and an optical measuring device for the PPG signal are required, such that it may be difficult to manufacture the measuring apparatus in a compact size. Further, both hands of a user may be required to contact the measuring apparatus to measure the ECG signal.

SUMMARY

One or more exemplary embodiments provide blood pressure measuring technology using a single light source, a plurality of light sources, and a light source selection process.

According to an aspect of an exemplary embodiment, there is provided a blood pressure measuring apparatus including: a light emitter configured to emit one or more lights having different penetration characteristics toward a user; light receiver configured to receive the lights that have penetrated through the user, and acquire photo-plethysmography (PPG) signals from the received lights; and a blood pressure measurer configured to measure a phase difference between the acquired PPG signals, and measure a blood pressure based on the measured phase difference.

The light emitter may include: a first light source configured to emit a first light in a range of an infrared wavelength to a red wavelength; and a second light source configured to emit a second light in a range of a blue wavelength to an ultraviolet wavelength.

The light emitter may further include a third light source configured to emit a third light in a range of a green wavelength.

The light receiver may acquire a first PPG signal from the first light, a second PPG signal from the second light, and a third PPG signal from the third light. The blood pressure measurer may measure a first phase difference between the first PPG signal and the second PPG signal, a second phase difference between the first PPG signal and the third PPG signal, and a third phase difference between the second PPG signal and the third PPG signal. The blood pressure measuring apparatus may calculate an average value of a first pulse wave velocity of the first PPG signal, a second pulse wave velocity of the second PPG signal, and a second pulse velocity of the third PPG signal based on the first phase difference, the second phase difference, and the third phase difference, and estimate a blood pressure based on the calculated average value.

The light emitter may include a single light source that emits a white light of a multi-wavelength band.

The light receiver may receive the white light penetrating though the user and filter the received white light by using a Red, Green and Blue (RGB) filter, and may acquire the PPG signal from the light filtered for each wavelength.

The light emitter may include, as the first light source, a light source that emits light having a first diffusion angle in a body of the user, and may include, as the second light source, a light source that emits a light having a second diffusion angle in the body. The second diffusion angle may be greater than the first diffusion angle.

The blood pressure measurer may calculate the pulse wave velocity based on the phase difference, and may estimate the blood pressure by using a relationship between the calculated pulse wave velocity and the blood pressure.

The blood pressure measuring apparatus may further include a pressure sensor configured to measure a tactile pressure input from the body, wherein the blood pressure measurer may further determine whether the measured tactile pressure is within a predetermined range of a tactile pressure.

The blood pressure measurer may determine a reference pressure within the predetermined range of the tactile pressure, and may calculate a correction coefficient for the measured tactile pressure based on the determined reference pressure.

The blood pressure measuring apparatus may further include a temperature sensor configured to measure a temperature of the body, wherein the blood pressure measurer may correct an error of the measured blood pressure based on the measured temperature and a relationship between the body temperature and the blood pressure.

According to an aspect of another exemplary embodiment, there is provided a blood pressure measuring apparatus using a light source selection process, the apparatus including: a light source array comprising a plurality of light sources; a processor configured to selectively turn on one or more light sources from among the plurality of light sources to emit one or more lights toward a user; a light receiver configured to receive the lights that have penetrated through the user, and acquire photo-plethysmography (PPG) signals from the received lights; and a blood pressure measurer configured to measure a phase difference between the acquired PPG signals, and measure a blood pressure based on the measured phase difference.

The processor may select a first light source and a second light receiver from among the plurality of lights sources of the light source array. The second light source may be disposed closer to the light receiver than the first light source.

The processor may correct the phase difference based on a time delay between the PPG signals.

The blood pressure measuring apparatus may further include a fingerprint recognition sensor configured to recognize fingerprints, wherein the processor may selectively turn on the one or more light sources based on at least one of a contact shape, a contact area, and a fingerprint pattern identified by the recognized fingerprints.

The processor may further provide the user with information about an appropriate contact position in which a finger of the user is to be placed to measure the blood pressure.

The processor may determine a predetermined position of a finger as a light emission position based on the fingerprint pattern, and may select the one or more light sources to emit the lights on the light emission position from among the plurality of light sources of the light source array.

The processor may determine the light emission position based on a location of a light source that maximizes the phase difference.

The processor may turn on a light source that is closer to the contact area than other light sources from among the plurality of light sources.

The processor may further include a storage configured to recognize individual users based on the recognized fingerprints, and store the measured blood pressure for the individual users.

The blood pressure measurer may calculate a pulse wave velocity based on the phase difference, and may estimate the blood pressure based on a relationship between the calculated pulse wave velocity and the blood pressure.

According to an aspect of another exemplary embodiment, there is provided a blood pressure measuring device including: a first light emitter configured to emit a first light of a first wavelength toward a subject; a second light emitter configured to emit a second light of a second wavelength that is shorter than the first wavelength toward the subject; a light detector configured to receive the first light passing through the subject after being emitted from the first light emitter and the second light passing through the subject after being emitted from the second light emitter; and a processor configured to detect a first photo-plethysmography (PPG) signal and a second PPG signal respectively from the received first light and the received second light, and determine a blood pressure of the subject based on a phase difference between the first PPG signal and the second PPG signal.

The blood pressure measuring device may further include a third light emitter configured to emit a third light of a third wavelength that is shorter than the first wavelength and longer than the second wavelength.

The second light emitter, the third light emitter, and the first light emitter may be respectively positioned in an order of increasing distance from the light detector.

The first light emitter, the second light emitter, the third light emitter correspond to a red light emitting diode (LED), a blue LED, and a green LED, respectively.

BRIEF DESCRIPTION OF THE DRAWINGS

The above and/or other aspects will be more apparent by describing certain exemplary embodiments, with reference to the accompanying drawings, in which:

FIG. 1 is a block diagram illustrating a blood pressure measuring apparatus according to an exemplary embodiment.

FIG. 2 is a diagram illustrating an example of a blood pressure measuring apparatus using a light source selection process according to another exemplary embodiment.

FIG. 3 is a diagram illustrating an example of emitting light on a finger.

FIG. 4 is a diagram illustrating an example of light, emitted from a light source, passing through a user's body part to be received by a light receiver.

FIG. 5 is a diagram illustrating an example of light, emitted from three light sources, passing through a user's body part to be received by a light receiver.

FIG. 6 is a diagram illustrating an example of penetration paths in a user's body of lights having different penetration characteristics.

FIG. 7 is a diagram illustrating an example of a filter layer of a PPG signal acquirer.

FIG. 8 is a diagram illustrating a graph of a phase difference between PPG signals.

FIG. 9 is a diagram illustrating a graph of a phase difference between three PPG signals.

FIG. 10 is a diagram illustrating an example of a blood pressure measuring apparatus further including a pressure sensor.

FIG. 11 is a diagram illustrating a graph showing a recommended range of a finger tactile pressure.

FIG. 12 is a diagram illustrating an example of a blood pressure measuring apparatus further including a transparent temperature sensor.

FIG. 13 is a diagram illustrating an example of a phase difference change depending on a temperature change.

FIG. 14 is a diagram illustrating an example of light, emitted from light sources that are located at different distances from a PPG signal acquirer, passing through a user's body to be received by a light receiver.

FIG. 15 is a diagram illustrating an example of selecting a light source located close to a contact area of a processor.

FIG. 16 is a diagram illustrating an example of measuring a phase difference between PPG signals.

FIG. 17 is a diagram illustrating graphs showing a phase difference at each wavelength.

DETAILED DESCRIPTION

Exemplary embodiments are described in greater detail below with reference to the accompanying drawings.

In the following description, like drawing reference numerals are used for like elements, even in different drawings. The matters defined in the description, such as detailed construction and elements, are provided to assist in a comprehensive understanding of the exemplary embodiments. However, it is apparent that the exemplary embodiments can be practiced without those specifically defined matters. Also, well-known functions or constructions are not described in detail since they would obscure the description with unnecessary detail.

Expressions such as "at least one of," when preceding a list of elements, modify the entire list of elements and do not modify the individual elements of the list.

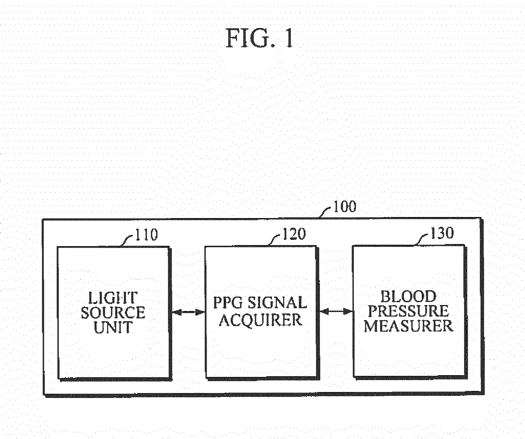

FIG. 1 is a block diagram illustrating a blood pressure measuring apparatus according to an exemplary embodiment. The blood pressure measuring apparatus 100 includes a light source unit 110, a photo-plethysmography (PPG) signal acquirer 120, and a blood pressure measurer 130. The light source unit 110 and the PPG signal acquirer 120 may be implemented by a light emitter and a light receiver (or a light detector), respectively, The light source unit 110 and the PPG signal acquire 120 may be integrated into a single light emitter/receiver, or may be provided with two separate devices. Further, the blood pressure measuring apparatus 100 may include a pressure sensor and a temperature sensor.

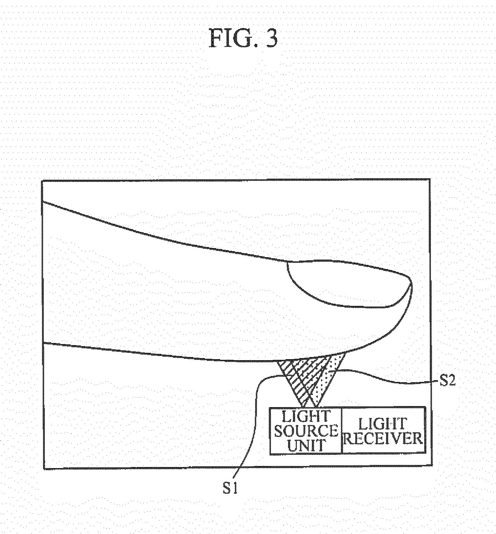

The light source unit 110 emits one or more lights having different characteristics of penetration into a user's body part, for example, a finger. Referring to FIG. 3, the light source unit 110 emits, onto a finger, a first light S1 and a second light S2 which have different penetration characteristics (e.g., penetration depth) from each other.

For example, light of different wavelengths may be transmitted to the body at different pulse wave velocities. In this case, the light source unit 110 includes a first light source, which emits the first light in a range of an infrared wavelength (e.g., 700 nm - 1 mm) to a red wavelength (e.g., 620-750 nm), and the second light source, which emits the second light in a range of a blue wavelength (e.g., 450-495 nm) to an ultraviolet wavelength (e.g., 10 nm — 400 nm). Further, the light source unit 110 may include a third light source that emits a third light in a range of a green wavelength (e.g., 495-570 nm). For example, the first light of a long wavelength, which is in a range of a red wavelength, may penetrate the skin and may reach, for example, blood vessels deep inside the skin, while the second light of a short wavelength may penetrate the skin to a shallow depth, to capillaries.

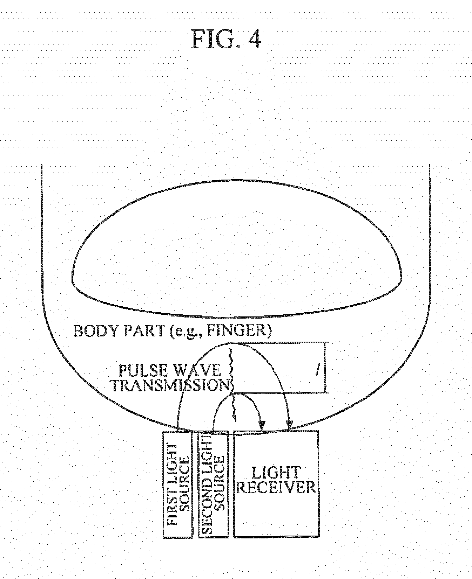

FIG. 4 is a diagram illustrating an example of light, emitted from a light source, passing through a user's body part to be received by a light receiver. Referring to FIG. 4, the first light source and the second light source each emit light on a user's body part, and the first light and the second light are transmitted through different paths in the body to the light receiver (e.g., PPG signal acquirer 120). Inside the body, the first light and the second light are spaced apart from each other at a distance (*l*). In this case, pulse wave velocities may vary depending on wavelength characteristics, and the first light and the second light, after passing through the body, may be received by the light receiver (e.g., PPG single acquirer 120) with a predetermined phase difference (PPT).

The first light is in a range of an infrared wavelength to a red wavelength, and has a long wavelength and a high pulse wave velocity. The second light is in a range of a blue wavelength to an ultraviolet wavelength, and has a lower pulse wave velocity than the first light. As the pulse wave velocity varies depending on wavelengths, light is received by the light receiver (e.g., PPG signal acquirer 120) with a phase difference.

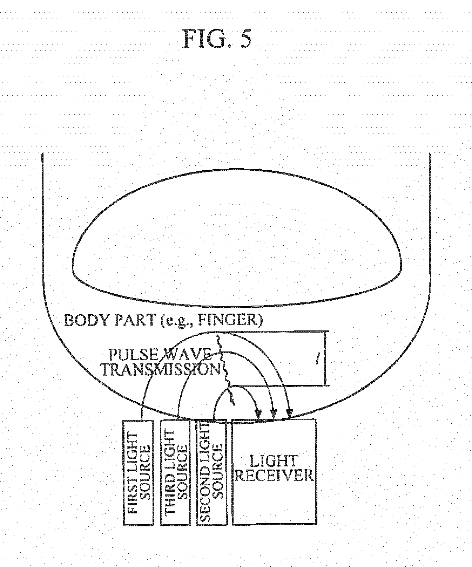

FIG. 5 is a diagram illustrating an example of light, emitted from three light sources, passing through a user's body part to be received by a light receiver. Referring to FIG. 5, a first light source, a second light source, and a third light source each emit light on a finger, and three emitted lights have different pulse wave velocities to penetrate into the skin to be received by the light receiver (e.g., PPG signal acquirer 120).

As an example of FIG. 5, the first light source of the light source unit 110 may emit a first light in a range of a red wavelength, the second light source of the light source unit 110 may emit a second light in a range of a blue wavelength, and the third light source of the light source unit 110 may emit a third light in a range of a green wavelength.

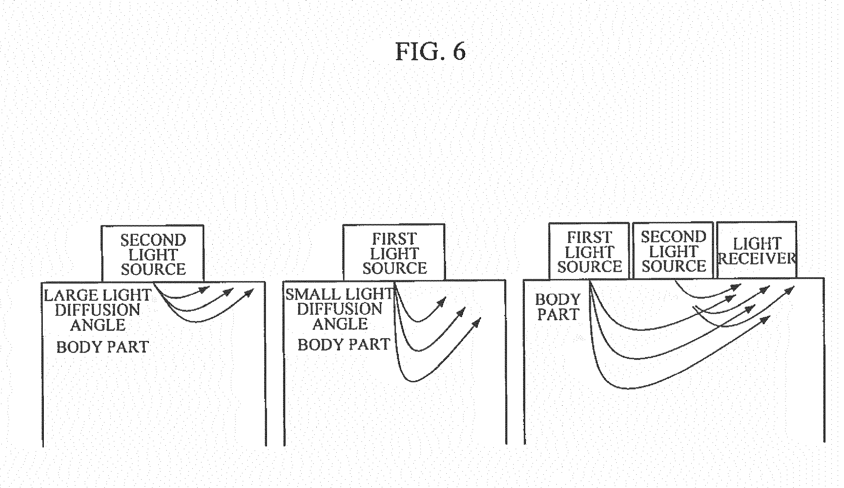

In another example, the light source unit 110 may include: as the first light source, a light source that emits light having a small diffusion angle in the body; and as the second light source, a light source that emits light having a larger diffusion angle in the body than the first light source. In this case, the light source, which emits light having a small diffusion angle, may be a laser diode (LD), and the light source, which emits light having a large diffusion angle, may be a light emitting diode (LED). FIG. 6 is a diagram illustrating an example of penetration paths in a user's body of lights having different penetration characteristics. The characteristics of light penetration into the body may include the length of a light wavelength, a diffusion angle of light, constituents according to the depth of body parts, light penetration speed, and the like.

The arrangement of the first light source, the second light source, and the third light source in relation to the light receiver may be determined based on the wavelength of each of the light sources. For example, the shorter the wavelength of the light source is, the closer the light source is positioned in relation to the light receiver. With reference to FIG. 5, the first light source, the second light source, and the third light source may correspond to a red LED, a blue LED, and a green LED, respectively, so that the blue LED, the green LED, and the red LED is disposed in the order of increasing their distance from the light receiver.

For example, the light source unit 110 may include a first light source and a second light source that have different diffusion angles in the body, in which a light source having a large diffusion angle may penetrate a shallow and wide part, while a light source having a small diffusion angle may penetrate a deep and narrow part.

Referring to FIG. 6, the light source unit 110 may include a laser diode (LD) and an LED as the first light source and the second light source, respectively. The diffusion angle of the LD is less than the diffusion angle of the LED. Each light emitted by the light source unit 110 penetrates a user's body through a different transmission path at a different penetration velocity, to be received by the light receiver (e.g., PPG signal acquirer 120). In another example, the light source unit 110 may emit white light including a multi-wavelength band. The white light is a single light and may include a multi-wavelength band, in which the PPG signal acquirer 120 may split the white light into different wavelengths of light.

The PPG signal acquirer 120 receives light that has passed through a user's body part, and may acquire a PPG signal from the received light. The PPG signal is a signal obtained by emitting light of a specific wavelength band on the body part and by detecting reflected or penetrated light, and a signal that indicates pulsation component generated according to a heartbeat rate.

For example, light emitted by the light source unit 110 is reflected from the body surface or passes through the body part, and is received by the light receiver of the PPG signal acquirer 120 with a phase difference. For example, a first light, which is emitted from the first light source and is in a range of a red wavelength, has a long transmission path in the body but a high pulse wave velocity, while a second light, which is emitted from the second light source and is in a range of a blue wavelength, has a short transmission path in the body but a low pulse wave velocity. The first light and the second light may be received by the PPG signal acquirer 120 with a phase difference.

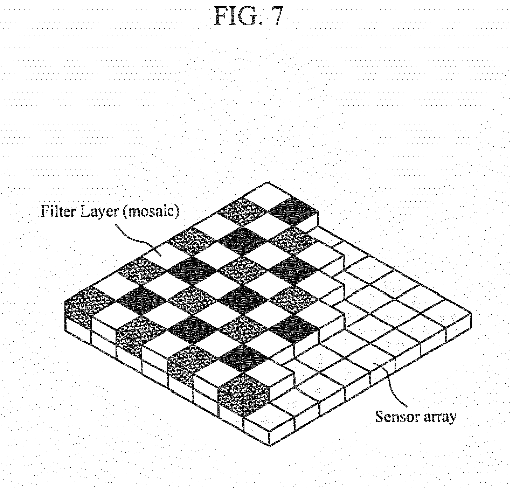

In another example, the PPG signal acquirer 120 may filter the single white light and pass only an allowed wavelength band of the light. FIG. 7 is a diagram illustrating an example of a filter layer of a PPG signal acquirer 120. Referring to FIG. 7, the PPG signal acquirer 120 may include an array of sensors, and a Red, Green and Blue (R-G-B) filter layer (mosaic). The RGB filter may filter light for red, green, and blue wavelengths. In this case, the PPG signal acquirer 120 may filter white light of a multi-wavelength band for each wavelength by using the RGB filter, and may acquire a PPG signal from each filtered light.

By using a single white light, and a single PPG signal acquirer 120, the blood pressure measuring apparatus 100 may be designed in a simple manner. The blood pressure measuring apparatus 100 of a simple design may be used in various applications, such as a smartphone, a tablet PC, a digital camera, a camera module, a wearable device, and the like.

The PPG signal acquirer 120 acquires a PPG signal from each received light. For example, the PPG signal acquirer 120 may receive a first PPG signal from the first light, a second PPG signal from the second light, a third PPG signal from the third light, and the like. The PPG signal acquirer 120 may receive a plurality of lights, and the number of which is not limited.

The PPG signal acquirer 120 may be a PPG sensor or an image sensor, and may also be a processor having a specific algorithm to acquire a PPG signal by processing a signal of the received light.

The blood pressure measurer 130 measures a phase difference (PPT) between feature points of acquired PPG signals, and may measure a blood pressure based on the measured phase difference. For example, the blood pressure measurer 130 may calculate a pulse wave velocity by extracting features points (e.g., peak points) by differentiating each PPG signal, and by measuring a phase difference (time difference) between the first PPG signal and the second PPG signal. Upon calculating the pulse wave velocity, the blood pressure measurer 130 may estimate a blood pressure based on a relationship between the pulse wave velocity and blood pressure. Blood pressure may be measured by such operations.

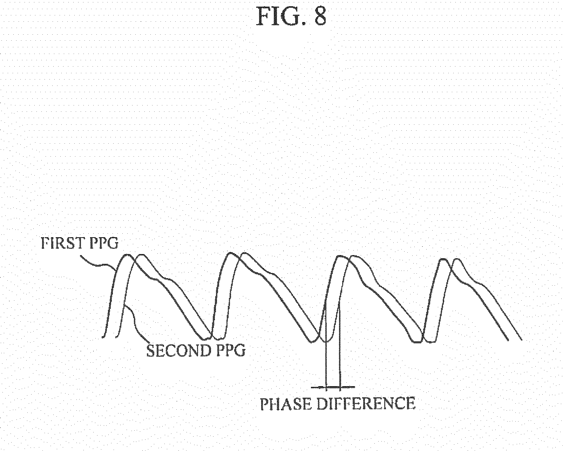

FIG. 8 is a diagram illustrating a graph of a phase difference between PPG signals. Referring to FIG. 8, a phase of the first PPG signal is acquired from light of a red wavelength and a phase of the second PPG signal is acquired from light of a blue wavelength that has a shorter wavelength than the red wavelength. The first PPG signal propagates faster than the second PPG signal. In other words, the phase velocity of the first PPG signal is greater than the phase velocity of the second PPG signal. In this case, the blood pressure measurer 130 may measure a phase difference between the first PPG signal and the second PPG signal.

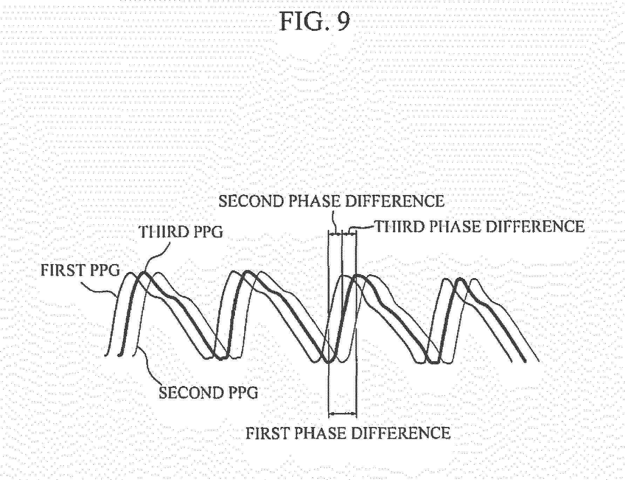

FIG. 9 is a diagram illustrating a graph of a phase difference between three PPG signals. In the exemplary embodiment, the blood pressure measurer 130 calculates a first pulse wave velocity based on a first phase difference between the first PPG signal and the second PPG signal; calculates a second pulse wave velocity based on a second phase difference between the first PPG signal and a third PPG signal; and calculates a third pulse wave velocity based on a third phase difference between the second PPG signal and the third PPG signal. The blood pressure measurer 130 may calculate an average of the three pulse wave velocities and may estimate a blood pressure based on the calculated average pulse wave velocity.

In addition, as the blood pressure measurer 130 may measure a phase difference among a plurality of PPG signals, and may calculate an average pulse wave velocity by using a plurality of phase differences, an error in measurement of phase difference may be reduced, and a blood pressure may be measured accurately.

Upon measuring a phase difference, the blood pressure measurer 130 may calculate a pulse wave velocity c by using the following Equation (1).

$$[\text{Equation 1}]$$

$$c = \frac{l}{\Delta T}$$

Here, length $l$ is a distance between the first PPG signal generator and the second PPG signal generator, and $\Delta T$ is a phase difference (time difference) between the first PPG signal and the second PPG signal. Since there is a direct relationship between a pulse wave velocity and a blood pressure, the blood pressure measurer 130 may estimate the blood pressure by calculating the pulse wave velocity.

In another example, the pulse wave velocity may be calculated by the following Equations (2) and (3).

$$[\text{Equation 2}]$$

$$E = E_0 e^{\alpha p}$$

$$[\text{Equation 3}]$$

$$c = \sqrt{\frac{Eh}{2\rho r}}$$

Here, E represents Young's modulus; $E_0$ represents Young's modulus at pressure 0; a represents a constant according to blood vessel characteristics; $p$ represents a blood pressure; c represents a pulse wave velocity; $h$ represents a blood vessel thickness; $p$ represents a density; and r represents a parameter based on a blood vessel radius. The pulse wave velocity c may be calculated by calculating or measuring these parameters.

Generally, when a blood pressure increases, the Young's modulus also increases, and the pulse wave velocity becomes faster. A PPG signal of the first light and a PPG signal of the second light have different penetration depths in the body, and when the PPG signals are measured at two specific points, a phase difference is caused between the two PPG signals.

The blood pressure measurer 130 may calculate the pulse wave velocity by using Equation (1) without applying the parameters of $p, h, \rho,$ and $r$ in the calculation. Upon calculating the pulse wave velocity, the blood pressure measurer 130 may determine the blood pressure based on a relationship between the pulse wave velocity and the blood pressure.

Further, the blood pressure measuring apparatus 100 illustrated in FIG. 1 may further include at least one or more of a pressure sensor and a temperature sensor.

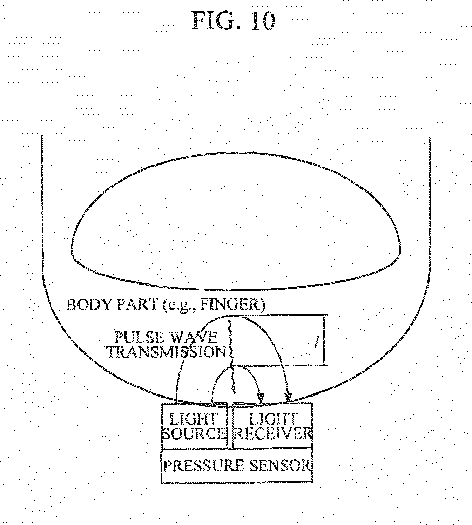

FIG. 10 is a diagram illustrating an example of a blood pressure measuring apparatus further including a pressure sensor. Referring to FIG. 10, once a user contacts a light source with his/her finger, the weight of the finger may be measured by a pressure sensor mounted at a lower portion of the light source and a light receiver. In this case, there may be a predetermined range of a tactile pressure that is appropriate for measurement of blood pressure, and the blood pressure measurer 130 may determine whether the measured pressure is within the predetermined range of the tactile pressure.

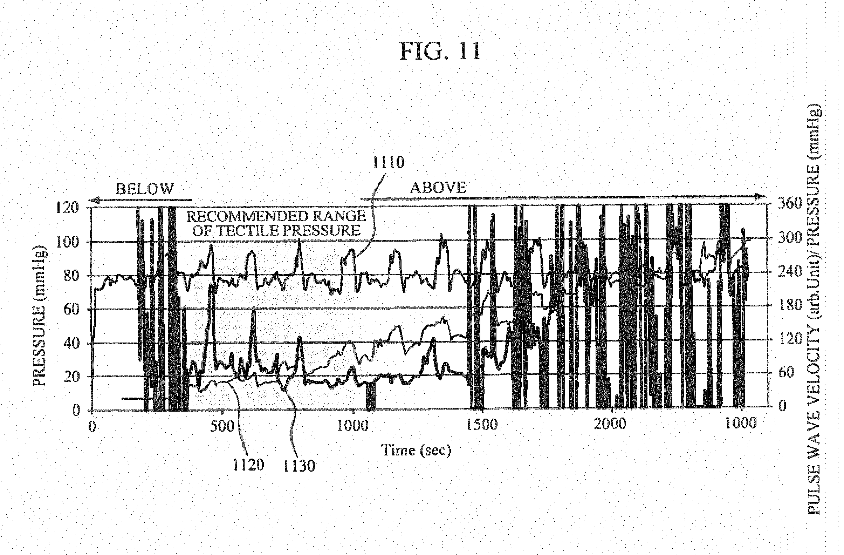

FIG. 11 is a diagram illustrating a graph showing a recommended range of a finger tactile pressure. The finger tactile pressure may be also referred to as a finger contact pressure. Referring to FIG. 11, when the pressure increases to reach a level that is below or above a predetermined range, the pulse wave velocity may not be calculated accurately. Generally, a pulse wave velocity graph and a blood pressure graph have the same shape and peak, and a blood pressure may be estimated by calculating a pulse wave velocity from the graphs. Referring to FIG. 11, however, as for levels of pressure that are not within the recommended range of the finger tactile pressure, the pulse wave velocities may show unstable peaks, rather than a predetermined shape of graph. That is, in the case where the measured pressure is not within the recommend range of the finger tactile pressure, the pulse wave velocity may not be calculated accurately, and a significant relationship between the pulse wave velocity and blood pressure may not be formed.

The blood pressure measurer 130 may determine whether a pressure, measured by the pressure sensor, is within the recommended range of the finger tactile pressure. When the measured pressure is beyond the recommended range, the blood pressure measuring apparatus 100 may generate a message that requests the user to adjust the finger tactile pressure. Further, even when the measured pressure is within the recommended range of the finger tactile pressure, a measurement error may be corrected according to an appropriate reference pressure. For example, the blood pressure measurer 130 may determine a reference pressure that is in the recommended range of the finger tactile pressure, and may calculate a correction factor for the measured pressure based on the determined reference pressure. For example, in the case where the measured pressure is greater than the reference pressure, the blood pressure measurer 130 may calculate a correction factor to correct the pulse wave velocity to be higher. By contrast, in the case where the measured pressure is less than the reference pressure, the blood pressure measurer 130 may calculate a correction factor to correct the pulse wave velocity to be lower. In this manner, the blood pressure measurer 130 may achieve an accurate blood pressure measurement regardless of a pressure exerted by a user. In the exemplary embodiment, a finger is used as a body part that may be in contact with the blood pressure measurer 130, but the present embodiment is not limited thereto. The user may use a different part of his/her body to contact the blood pressure measurer 130 and the contact pressure may be compared with a predetermined pressure range.

The blood pressure measuring apparatus 100 may further include an interface or an application, which informs a user of a finger tactile pressure measured by a pressure sensor, and provides an alarm function to a user so that an appropriate pressure may be input.

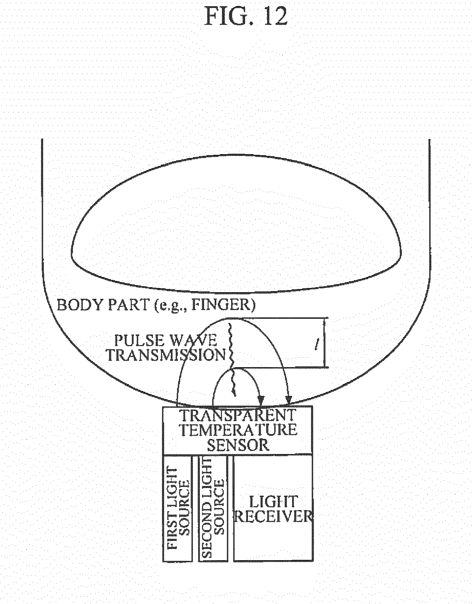

FIG. 12 is a diagram illustrating an example of a blood pressure measuring apparatus 100 which further includes a transparent temperature sensor that may measure the body temperature. Referring to FIG. 12, the transparent temperature sensor is disposed on the upper portion of a light source and a light receiver, to measure the body temperature without blocking light emitted from the light source.

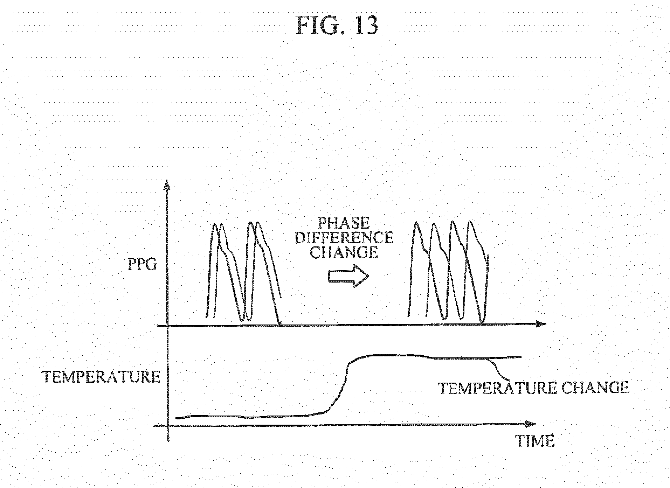

FIG. 13 is a diagram illustrating an example of a phase difference change depending on a temperature change. Referring to FIG. 13, the phase difference between the first PPG signal and the second PPG signal at a high temperature is greater than the phase difference at a low temperature. Once the temperature of a finger is measured by a temperature sensor 150, the blood pressure measurer 130 may correct an error of phase difference based on the measured temperature.

By using at least one of a pressure sensor and a temperature sensor, the blood pressure measuring apparatus 100 may check factors that affect a measurement result of a blood pressure, and may correct an error in the measurement result of the blood pressure.

The blood pressure measuring apparatus 100 may be included in a digital camera, an image sensor of a camera module, and the like, and may also be mounted in a smartphone, a tablet PC, a wearable device, a healthcare product, and the like.

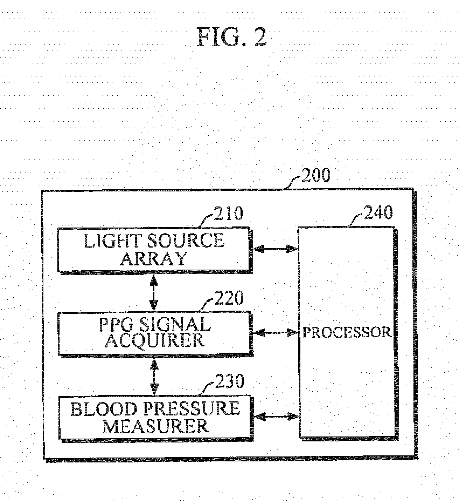

FIG. 2 is a diagram illustrating an example of a blood pressure measuring apparatus using a light source selection process according to another exemplary embodiment. The blood pressure measuring apparatus 200 using a light source selection process includes a light source array 210, a PPG signal acquirer 220, a blood pressure measurer 230, and a processor 240. The blood pressure measurer 230 may be integrated with the processor 240, or another processor separately provided from the processor 240. Further, the blood pressure measuring apparatus 200 using a light source selection process may further include a fingerprint recognition sensor, a pressure sensor, and a temperature sensor, in which elements that overlap with those illustrated in FIG. 1 will be briefly described hereinafter.

The light source array 210 may include a plurality of light sources, including a fist light source, a second light source, a third light source, and the like, which have different wavelengths. Further, the light source array 210 may include a laser diode and a light emitting diode (LED) which have different penetration characteristics in the body. In addition, the light source array 210 may include a plurality of single light sources, each emitting a single light such as white light.

The light source array 210 may arrange a plurality of light sources in a predetermined form or array. However, the arrangement form or array of the plurality of light sources in the light source array 210 is not limited, and there may be various array forms.

A processor 240 selects one or more light sources from among the plurality of light sources of the light source array 210, and may control the selected one or more light sources to be emitted on the body. Based on different penetration characteristics, the processor 240 may select one or more light sources by differing any one of a range of wavelengths, a range of diffusion angles, types of light sources, and locations of light sources.

For example, among the light sources of the light source array 210, the processor 240 may select the first light source located far from the PPG signal acquirer 220, or may select the second light source located closer to the PPG signal acquirer 220 than the first light source. The wavelength of the first light source may be greater than the wavelength of the second light source.

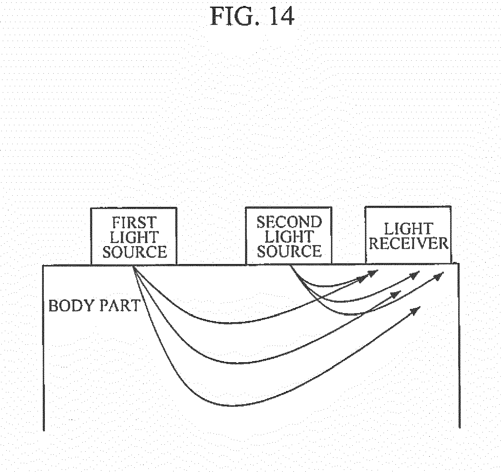

FIG. 14 is a diagram illustrating an example of light, emitted from light sources that are located at different distances from a PPG signal acquirer 220, passing through a user's body to be received by a light receiver. Referring to FIG. 14, the first light source, which is relatively farther from the PPG signal acquirer 220 (e.g., a light receiver), has a long transmission path in the body. Similarly, the second light source, which is relatively closer to the PPG signal acquirer 220, has a short transmission path. That is, depending on the location of light sources, there may be a phase difference between the first light source and the second light source that are received by the PPG signal acquirer 220 (e.g., the light receiver).

For example, the processor 240 may select the first light source, which is far from the PPG signal acquirer 220, and the second light source, which is close to the PPG signal acquirer 220. Referring to FIG 14, the distance between the first light source and the PPG signal acquirer 220 is longer than the distance between the second light source and the PPG signal acquirer 220, which may delay time for the first light to reach the light receiver. In this case, considering the time delay caused by different distances from the PPG signal acquirer 220, the processor 240 may correct a phase difference between the PPG signals.

Further, the blood pressure measuring apparatus 200 using a light source selection process may further include a fingerprint recognition sensor that recognizes fingerprints, in which the processor 240 may select a light source based on at least one of a contact shape, a contact area, and a fingerprint pattern, which are identified by the recognition of fingerprints.

For example, once the fingerprint recognition sensor recognizes a user's fingerprints, the processor 240 analyzes a contact shape, a contact area, and a fingerprint pattern, which are identified by the fingerprint recognition, and may identify a contact position of a finger.

In this case, the processor 240 may provide a user with information on an appropriate contact position of a finger, which is required to measure blood pressure. For example, the processor 240 may provide a user with the contact position of a finger, which is identified by fingerprint recognition, and a pre-stored appropriate contact position of a finger, which is required to measure blood pressure, through an interface. Further, the processor 240 may provide guide information to guide a user's finger to an appropriate position to measure blood pressure.

Among the light sources of the light source array 210, the processor 240 may select a light source that is located at an optimal location to emit light on the identified contact position of a finger.

For example, based on a finger pattern, the processor 240 may determine a position of a finger to be a light emission position, and may select a light source to emit light on the position from among the light sources of the light source array 210. For example, the processor 240 may determine a light emission position based on a location of a light source that maximizes a phase difference, in which the phase difference may be measured by experiments or by repetition, or may be calculated based on a location of a light source.

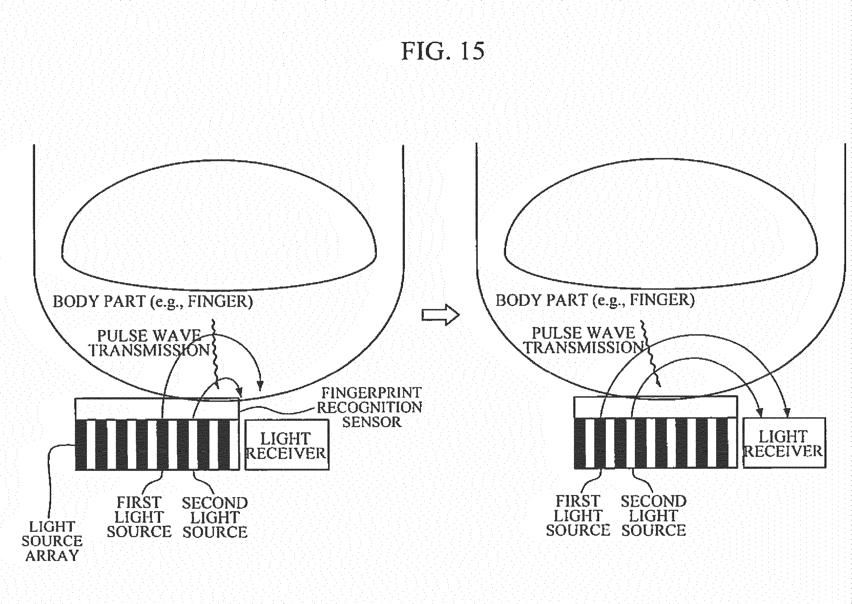

In another example, the processor 240 may select a light source that is close to a contact area. FIG. 15 is a diagram illustrating an example of selecting a light source located close to a contact area of a processor. Referring to FIG. 15, the light source array 210 may select two or more light sources having a wide contact area for a finger, and the processor 240 may select two or more light sources appropriately from among the light sources of the light source array 210.

In addition, the processor 240 may include a storage that recognizes individual users by recognition of fingerprints, and stores the measured blood pressure for each individual user.

The PPG signal acquirer 220 may receive light that has passed through the body part, and may acquire a Photo-plethysmography (PPG) signal from the received light.

The blood pressure measurer 230 may measure a phase difference between feature points of the acquired PPG signals, and may measure blood pressure based on the phase difference. For example, the blood pressure measurer 230 may calculate a pulse wave velocity by extracting features points (e.g., peak points) by differentiating each PPG signal, and by measuring a phase difference (time difference) between the first PPG signal and the second PPG signal. Upon calculating the pulse wave velocity, the blood pressure measurer 230 may estimate a blood pressure based on a relationship between the pulse wave velocity and the blood pressure. Blood pressure may be measured by such operations.

The blood pressure measuring apparatus 200 illustrated in FIG. 2 may identify states and conditions of blood pressure measurement by checking one or more of a fingerprint recognition sensor, a pressure sensor, and a temperature sensor, and may guarantee objectivity of blood pressure measurement.

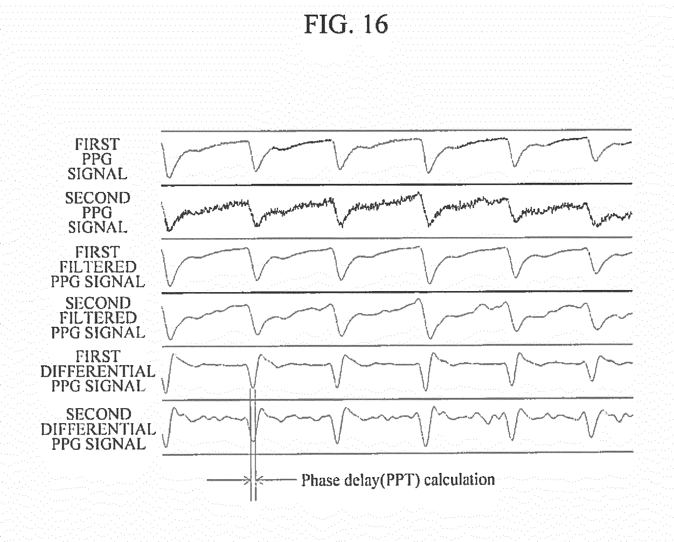

FIG. 16 is a diagram illustrating an example of measuring a phase difference between PPG signals. The blood pressure measurers 130 and 230 illustrated in FIGS. 1 and 2 may extract feature points from PPG signals, and may measure a phase difference between the feature points. For example, the blood pressure measurers 130 and 230 perform resampling of each PPG signal, for example, resampling of a frequency of 250 Hz to 2500 Hz, and pass the PPG signal through a low pass filter (LPF) to pass a frequency of lower than 10 Hz.

Referring to FIG. 16, the blood pressure measurers 130 and 230 filter graphs of the first PPG signal and the second PPG signal through the LPF, and may acquire the first and second filtered PPG signals. The first and second filtered PPG signals become graphs in a smoother shape than graphs before filtering. Further, the blood pressure measurers 130 and 230 extract feature points from graphs obtained by differentiating the first filtered PPG signal and the second filtered PPG signal. Upon extracting the feature points, the blood pressure measurers 130 and 230 may measure a phase difference between the first PPG signal and the second PPG signal.

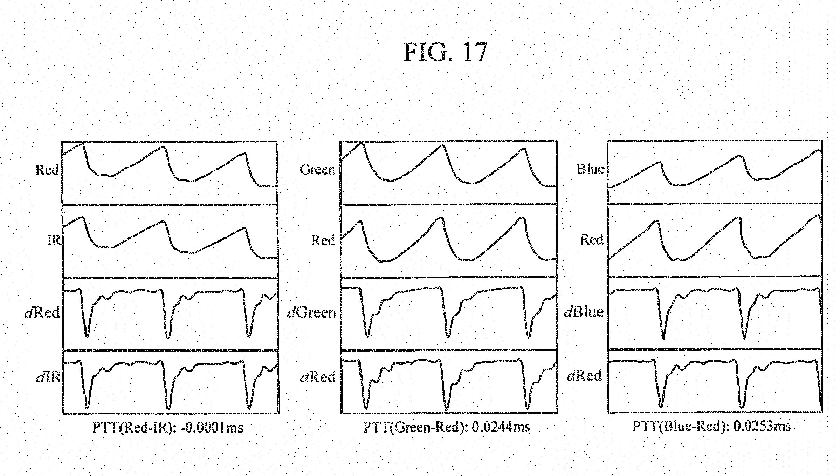

FIG. 17 is a diagram illustrating a graph showing a phase difference at each wavelength. Referring to FIG. 17, graphs show a phase difference between a red wavelength and an infrared wavelength, a phase difference between a red wavelength and a green wavelength, and a phase difference between a red wavelength and a blue wavelength. The blood pressure measurers 130 and 230 may extract feature points of each graph by differentiating each graph, and may measure a phase difference based on the feature points of each graph.

Referring to the left portion of FIG. 17, a phase difference between feature points of a differential graph (dRed) of a red wavelength and a differential graph (dIR) of an infrared wavelength is -0.0001 ms. In another example, referring to the middle portion of FIG. 17, a phase difference between feature points of a differential graph (dGreen) of a green wavelength and a differential graph (dRed) of a red wavelength is 0.0244 ms. In yet another example, referring to the right portion of FIG. 17, a phase difference between feature points of a differential graph (dBlue) of a blue wavelength and a differential graph (dRed) of a red wavelength is 0.0253 ms.

However, the above examples are merely illustrative, and red, blue and green wavelengths are selected randomly, and blood pressure may be measured by using other ranges of wavelengths.

According to the foregoing exemplary embodiment, a blood pressure measuring apparatus may have a simple configuration, and may minimize errors in the measurement of blood pressure in various circumstances.

While not restricted thereto, the operations or steps of the methods or algorithms according to the above exemplary embodiments may be embodied as computer-readable codes on a computer-readable recording medium. The computer-readable recording medium may be any recording apparatus capable of storing data that is read by a computer system. Examples of the computer-readable recording medium include read-only memories (ROMs), random-access memories (RAMs), CD-ROMs, magnetic tapes, floppy disks, and optical data storage devices. The computer-readable recording medium may be a carrier wave that transmits data via the Internet, for example. The computer-readable medium may be distributed among computer systems that are interconnected through a network so that the computer-readable code is stored and executed in a distributed fashion. Also, the operations or steps of the methods or algorithms according to the above exemplary embodiments may be written as a computer program transmitted over a computer-readable transmission medium, such as a carrier wave, and received and implemented in general-use or special-purpose digital computers that execute the programs. Moreover, it is understood that in exemplary embodiments, one or more units of the above-described apparatuses and devices can include or implemented by circuitry, a processor, a microprocessor, etc., and may execute a computer program stored in a computer-readable medium.

**Claims**

1. A blood pressure measuring apparatus comprising:

a light emitter configured to emit one or more lights having different penetration characteristics toward a user, the light emitter comprising a light source array (210) comprising a plurality of light sources;
a light receiver (220) configured to receive the lights that have penetrated through the user, and acquire photo-plethysmography (PPG) signals from the received lights; and
a blood pressure measurer (230) configured to measure a phase difference between the acquired PPG signals, and measure a blood pressure based on the measured phase difference, and
a processor (240) configured to selectively turn on one or more light sources from among the plurality of light sources to emit the one or more lights toward a user, the processor further being configured to select a first light source and a second light source from among the plurality of lights sources of the light source array, and wherein the second light source is disposed closer to the light receiver than the first light source, the processor further being configured to correct the phase difference based on a time delay between the PPG signals.

2. The apparatus of claim 1, wherein the light emitter comprises:

a first light source configured to emit a first light in a range of an infrared wavelength to a red wavelength; and
a second light source configured to emit a second light in a range of a blue wavelength to an ultraviolet wavelength.

3. The apparatus of claim 2, wherein the light emitter comprises a third light source configured to emit a third light in a range of a green wavelength, in particular, wherein:

the light receiver acquires a first PPG signal from the first light, a second PPG signal from the second light, and a third PPG signal from the third light;
the blood pressure measurer measures a first phase difference between the first PPG signal and the second PPG signal, a second phase difference between the first PPG signal and the third PPG signal, and a third phase difference between the second PPG signal and the third PPG signal,
wherein the blood pressure measuring apparatus calculates an average value of a first pulse wave velocity of the first PPG signal, a second pulse wave velocity of the second PPG signal, and a third pulse velocity of the third PPG signal based on the first phase difference, the second phase difference, and the third phase difference, and estimates a blood pressure based on the calculated average value.

4. The apparatus of claim 1, wherein the light emitter comprises a single light source that emits a white light of a multi-wavelength band.

5. The apparatus of claim 4, wherein the light receiver is further configured to receive the white light penetrating though the user, filter the received white light by using a Red, Green and Blue (RGB) filter, and acquire the PPG signal from the light filtered for each wavelength.

6. The apparatus of claim 2, wherein the light emitter comprises, as the first light source, a light source that emits a light having a first diffusion angle in a body of the user, and comprises, as the second light source, a light source that emits a light having a second diffusion angle in the body, the second diffusion angle being greater than the first diffusion angle.

7. The apparatus of claim 1, wherein the blood pressure measurer calculates a pulse wave velocity of the PPG signals based on a phase difference between the PPG signals, and estimates the blood pressure based on a relationship between the calculated pulse wave velocity and the blood pressure.

8. The apparatus of claim 1, further comprising a pressure sensor configured to measure a tactile pressure input from the body, wherein the blood pressure measurer further determines whether the measured tactile pressure is within a predetermined range of a tactile pressure, in particular, wherein the blood pressure measurer determines a reference pressure within the predetermined range of the tactile pressure, and calculates a correction coefficient for the measured tactile pressure based on the determined reference pressure.

9. The apparatus of claim 1, further comprising a temperature sensor configured to measure a temperature of the body, wherein the blood pressure measurer corrects an error of the measured blood pressure based on the measured temperature and a relationship between the body temperature and the blood pressure.

10. The apparatus of any one of claims 1 to 9, wherein the blood pressure measurer calculates a pulse wave velocity based on the phase difference, and estimates the blood pressure based on a relationship between the calculated pulse wave velocity and the blood pressure.

11. The apparatus of claim any one of claims 1 to 9, further comprising a fingerprint recognition sensor configured to recognize fingerprints, wherein the processor is further configured to selectively turn on the one or more light sources based on at least one of a contact shape, a contact area, and a fingerprint pattern identified by the recognized fingerprints.

12. The apparatus of claim 11,

wherein the processor is further configured to provide the user with information about an appropriate contact position in which a finger of the user is to be placed to measure the blood pressure, and/or
wherein the processor is further configured to turn on a light source that is closer to the contact area than other light sources from among the plurality of light sources, and/or
wherein the processor comprises a storage configured to recognize individual users based on the recognized fingerprints, and store the measured blood pressure for the individual users, and/or
wherein the processor is further configured to determine a predetermined position of a finger as a light emission position based on the fingerprint pattern and turn on the one or more light sources to emit the lights on the light emission position from among the plurality of light sources of the light source array,
in particular, wherein the processor is further configured to determine the light emission position based on a location of a light source that maximizes the phase difference.

13. The apparatus of claim 1,

wherein the one or more lights comprise a first light of a first wavelength and a second light of a second wavelength that is shorter than the first wavelength;
wherein the PPG signals comprise a first photoplethysmography (PPG) signal and a second PPG signal respectively from the received first light and the received second light; and
wherein the phase difference is a phase difference between the first PPG signal and the second PPG signal.

14. The blood pressure measuring device of claim 13, wherein the one or more lights further comprise a third light of a third wavelength that is shorter than the first wavelength and longer than the second wavelength,

in particular,

wherein the first light is emitted by a first light emitter, the second light is emitted by a second light emitter, and the third light is emitted by a third light emitter, and

wherein the second light emitter, the third light emitter, and the first light emitter are respectively positioned in an order of increasing distance from the light detector, and/or

wherein the first light emitter, the second light emitter, the third light emitter correspond to a red light emitting diode (LED), a blue LED, and a green LED, respectively.

**Patentansprüche**

1. Blutdruck-Messvorrichtung, umfassend:

einen Lichtsender, ausgebildet zum Ausstrahlen von einem oder mehreren Lichtstrahlen mit verschiedenen Durchdringungscharakteristiken zu einem Benutzer, wobei der Lichtsender eine Lichtquellengruppe (210), umfassend eine Vielzahl von Lichtquellen, umfasst; einen Lichtempfänger (220), ausgebildet zum Empfangen der Lichtstrahlen, die durch den Benutzer durchgedrungen sind, und Erfassen von Photo-Plethysmography-(PPG-) Signalen aus den empfangenen Lichtstrahlen; und einen Blutdruckmesser (230), ausgebildet zum Messen eines Phasenunterschieds zwischen den erfassten PPG-Signalen und Messen eines Blutdrucks auf der Basis des gemessenen Phasenunterschieds, und einen Prozessor (240), ausgebildet zum selektiven Einschalten von einer oder mehreren Lichtquellen aus der Vielzahl von Lichtquellen zum Ausstrahlen des einen oder der mehreren Lichtstrahlen zu einem Benutzer, wobei der Prozessor ferner zum Auswählen einer ersten Lichtquelle und einer zweiten Lichtquelle aus der Vielzahl von Lichtquellen der Lichtquellengruppe ausgebildet ist und wobei die zweite Lichtquelle näher am Lichtempfänger angeordnet ist als die erste Lichtquelle, wobei der Prozessor ferner zum Korrigieren des Phasenunterschieds auf der Basis einer Zeitverzögerung zwischen den PPG-Signalen ausgebildet ist.

2. Vorrichtung nach Anspruch 1, wobei der Lichtsender umfasst:

eine erste Lichtquelle, ausgebildet zum Senden eines ersten Lichtstrahls in einem Bereich einer Infrarot-Wellenlänge zu einer Rot-Wellenlänge; und

eine zweite Lichtquelle, ausgebildet zum Senden eines zweiten Lichtstrahls in einem Bereich einer Blau-Wellenlänge zu einer Ultraviolett-Wellenlänge.

3. Vorrichtung nach Anspruch 2, wobei der Lichtsender insbesondere eine dritte Lichtquelle, ausgebildet zum Senden eines dritten Lichtstrahls in einem Bereich einer Grün-Wellenlänge, umfasst, wobei:

der Lichtempfänger ein erstes PPG-Signal aus dem ersten Lichtstrahl, ein zweites PPG-Signal aus dem zweiten Lichtstrahl und ein drittes PPG-Signal aus dem dritten Lichtstrahl erfasst; der Blutdruckmesser einen ersten Phasenunterschied zwischen dem ersten PPG-Signal und dem zweiten PPG-Signal, einen zweiten Phasenunterschied zwischen dem ersten PPG-Signal und dem dritten PPG-Signal und einen dritten Phasenunterschied zwischen dem zweiten PPG-Signal und dem dritten PPG-Signal misst, wobei die Blutdruck-Messvorrichtung einen Durchschnittswert einer ersten Pulswellengeschwindigkeit des ersten PPG-Signals, eine zweite Pulswellengeschwindigkeit des zweiten PPG-Signals und eine dritte Pulsgeschwindigkeit des dritten PPG-Signals auf der Basis des ersten Phasenunterschieds, des zweiten Phasenunterschieds und des dritten Phasenunterschieds berechnet und einen Blutdruck auf der Basis des berechneten Durchschnittswerts schätzt.

4. Vorrichtung nach Anspruch 1, wobei der Lichtsender eine einzelne Lichtquelle umfasst, die einen weißen Lichtstrahl eines Mehrfach-Wellenlängen-Bandes ausstrahlt.

5. Vorrichtung nach Anspruch 4, wobei der Lichtempfänger ferner zum Empfangen des durch den Benutzer durchdringenden weißen Lichtstrahls, Filtern des empfangenen weißen Lichtstrahls durch Verwenden eines Rot-, Grün- und Blau-(RGB-)Filters und Erfassen des PPG-Signals aus dem für jede Wellenlänge gefilterten Lichts ausgebildet ist.

6. Vorrichtung nach Anspruch 2, wobei der Lichtsender als erste Lichtquelle eine Lichtquelle umfasst, die einen Lichtstrahl mit einem ersten Diffusionswinkel in einem Körper des Benutzers ausstrahlt, und als zweite Lichtquelle eine Lichtquelle umfasst, die einen Lichtstrahl mit einem zweiten Diffusionswinkel im Körper ausstrahlt, wobei der zweite Diffusionswinkel größer ist als der erste Diffusionswinkel.

7. Vorrichtung nach Anspruch 1, wobei der Blutdruckmesser eine Pulswellengeschwindigkeit der PPG-Signale auf der Basis eines Phasenunterschieds

**EP 3 153 094 B1**

zwischen den PPG-Signalen berechnet und den Blutdruck auf der Basis einer Beziehung zwischen der berechneten Pulswellengeschwindigkeit und dem Blutdruck schätzt.

8. Vorrichtung nach Anspruch 1, ferner umfassend einen Drucksensor, ausgebildet zum Messen einer taktilen Druckeingabe vom Körper,

   wobei der Blutdruckmesser ferner bestimmt, ob sich der gemessene taktile Druck innerhalb eines vorbestimmten Bereichs eines taktilen Drucks befindet,
   wobei der Blutdruckmesser insbesondere einen Referenzdruck im vorbestimmten Bereich des taktilen Drucks bestimmt und einen Korrekturkoeffizienten für den gemessenen taktilen Druck auf der Basis des bestimmten Referenzdrucks berechnet.

9. Vorrichtung nach Anspruch 1, ferner umfassend einen Temperatursensor, ausgebildet zum Messen einer Temperatur des Körpers,
   wobei der Blutdruckmesser einen Fehler des gemessenen Blutdrucks auf der Basis der gemessenen Temperatur und einer Beziehung zwischen der Körpertemperatur und dem Blutdruck korrigiert.

10. Vorrichtung nach einem der Ansprüche 1 bis 9, wobei der Blutdruckmesser eine Pulswellengeschwindigkeit auf der Basis des Phasenunterschieds berechnet und den Blutdruck auf der Basis einer Beziehung zwischen der berechneten Pulswellengeschwindigkeit und dem Blutdruck schätzt.

11. Vorrichtung nach einem der Ansprüche 1 bis 9, ferner umfassend einen Fingerabdruck-Erkennungssensor, ausgebildet zum Erkennen von Fingerabdrücken,
    wobei der Prozessor ferner zum selektiven Einschalten der einen oder mehreren Lichtquellen auf der Basis von wenigstens einem Element der Gruppe umfassend eine Kontaktform, eine Kontaktfläche und ein Fingerabdrucksmuster, identifiziert durch die erkannten Fingerabdrücke, ausgebildet ist.

12. Vorrichtung nach Anspruch 11,

    wobei der Prozessor ferner zum Versorgen des Benutzers mit Informationen zu einer geeigneten Kontaktposition, in der ein Finger des Benutzers zum Messen des Blutdrucks anzuordnen ist, ausgebildet ist, und/oder
    wobei der Prozessor ferner zum Einschalten einer Lichtquelle, die näher an der Kontaktfläche ist als andere Lichtquellen aus der Vielzahl von Lichtquellen, ausgebildet ist, und/oder wobei der Prozessor einen Speicher, ausgebildet zum Erkennen von einzelnen Benutzern auf der Basis der erkannten Fingerabdrücke und Speichern des gemessenen Blutdrucks für die einzelnen Benutzer, umfasst, und/oder wobei der Prozessor ferner zum Bestimmen einer vorbestimmten Position eines Fingers als eine Lichtausstrahlungsposition auf der Basis des Fingerabdruckmusters und Einschalten der einen oder mehreren Lichtquellen zum Ausstrahlen der Lichtstrahlen in der Lichtausstrahlungsposition aus der Vielzahl von Lichtquellen der Lichtquellengruppe ausgebildet ist,
wobei der Prozessor insbesondere ferner zum Bestimmen der Lichtausstrahlungsposition auf der Basis einer Anordnung einer Lichtquelle, die den Phasenunterschied maximiert, ausgebildet ist.

13. Vorrichtung nach Anspruch 1,

    wobei der eine oder die mehreren Lichtstrahlen einen ersten Lichtstrahl einer ersten Wellenlänge und einen zweiten Lichtstrahl einer zweiten Wellenlänge, die kürzer ist als die erste Wellenlänge, umfassen;
    wobei die PPG-Signale ein erstes Photo-Plethysmography-(PPG-) Signal und ein zweites PPG-Signal jeweils vom empfangenen ersten Lichtstrahl und empfangenen zweiten Lichtstrahl umfassen; und wobei der Phasenunterschied ein Phasenunterschied zwischen dem ersten PPG-Signal und dem zweiten PPG-Signal ist.

14. Blutdruck-Messvorrichtung nach Anspruch 13, wobei der eine oder die mehreren Lichtstrahlen einen dritten Lichtstrahl einer dritten Wellenlänge, die kürzer als die erste Wellenlänge und länger als die zweite Wellenlänge ist, umfassen,

    wobei insbesondere der erste Lichtstrahl von einem ersten Lichtsender ausgestrahlt wird, der zweite Lichtstrahl von einem zweiten Lichtsender ausgestrahlt wird und der dritte Lichtstrahl von einem dritten Lichtsender ausgestrahlt wird, und
    wobei der zweite Lichtsender, der dritte Lichtsender und der erste Lichtsender jeweils in einer Reihenfolge zunehmenden Abstands zum Lichtdetektor angeordnet sind, und/oder wobei der erste Lichtsender, der zweite Lichtsender und der dritte Lichtsender jeweils einer roten LED, einer blauen LED und einer grünen LED entsprechen.

**Revendications**

1. Appareil de mesure de la tension artérielle comprenant :

     un émetteur de lumière conçu pour émettre une ou plusieurs lumières ayant différentes caractéristiques de pénétration vers un utilisateur, l'émetteur de lumière comprenant un réseau de sources de lumière (210) comprenant une pluralité de sources de lumière ;

     un récepteur de lumière (220) conçu pour recevoir les lumières qui ont pénétré à travers l'utilisateur, et pour acquérir des signaux de photopléthysmographie (PPG) de la part des lumières reçues ; et

     un dispositif de mesure de tension artérielle (230) conçu pour mesurer une différence de phase entre les signaux PPG acquis, et pour mesurer une tension artérielle sur la base de la différence de phase mesurée, et

     un processeur (240) conçu pour mettre en marche sélectivement une ou plusieurs sources de lumière parmi la pluralité des sources de lumière afin d'émettre lesdites lumières vers un utilisateur,

     le processeur étant en outre conçu pour sélectionner une première source de lumière et une seconde source de lumière à partir de la pluralité des sources de lumière du réseau de sources de lumière, et la seconde source de lumière étant disposée plus près du récepteur de lumière que la première source de lumière, le processeur étant en outre conçu pour corriger la différence de phase sur la base d'un retard de temps entre les signaux PPG.

2. Appareil selon la revendication 1, l'émetteur de lumière comprenant :

     une première source de lumière conçue pour émettre une première lumière dans une plage d'une longueur d'onde infrarouge à une longueur d'onde rouge ; et

     une seconde source de lumière conçue pour émettre une seconde lumière dans une plage d'une longueur d'onde bleue à une longueur d'onde ultraviolette.

3. Appareil selon la revendication 2, l'émetteur de lumière comprenant une troisième source de lumière conçue pour émettre une troisième lumière dans une plage d'une longueur d'onde verte, en particulier :

     le récepteur de lumière acquérant un premier signal PPG de la part de la première lumière, un second signal PPG de la part de la seconde lumière, et un troisième signal PPG de la part de la troisième lumière ;

     le dispositif de mesure de tension artérielle mesurant une première différence de phase entre le premier signal PPG et le second signal PPG, une seconde différence de phase entre le premier signal PPG et le troisième signal PPG, et une troisième différence de phase entre le second signal PPG et le troisième signal PPG, l'appareil de mesure de la tension artérielle calculant une valeur moyenne d'une première vitesse d'onde d'impulsion du premier signal PPG, d'une seconde vitesse d'onde d'impulsion du second signal PPG, et d'une troisième vitesse d'impulsion du troisième signal PPG sur la base de la première différence de phase, de la seconde différence de phase, et de la troisième différence de phase, et estimant une tension artérielle sur la base de la valeur moyenne calculée.

4. Appareil selon la revendication 1, l'émetteur de lumière comprenant une source de lumière unique qui émet une lumière blanche d'une bande de multiples longueurs d'ondes.

5. Appareil selon la revendication 4, le récepteur de lumière étant en outre conçu pour recevoir la lumière blanche pénétrant à travers l'utilisateur, filtrer la lumière blanche reçue en utilisant un filtre rouge, vert et bleu (RGB), et acquérir le signal PPG à partir de la lumière filtrée pour chaque longueur d'onde.

6. Appareil selon la revendication 2, l'émetteur de lumière comprenant, comme première source de lumière, une source de lumière qui émet une lumière ayant un premier angle de diffusion dans un corps de l'utilisateur, et qui comprend, comme seconde source de lumière, une source de lumière qui émet une lumière ayant un second angle de diffusion dans le corps, le second angle de diffusion étant supérieur au premier angle de diffusion.

7. Appareil selon la revendication 1, le dispositif de mesure de tension artérielle calculant une vitesse d'onde d'impulsion des signaux PPG sur la base d'une différence de phase entre les signaux PPG, et estimant la tension artérielle sur la base d'une relation entre la vitesse d'onde d'impulsion calculée et la tension artérielle.

8. Appareil selon la revendication 1, comprenant en outre un capteur de pression conçu pour mesurer une entrée de pression tactile depuis le corps, le dispositif de mesure de tension artérielle déterminant en outre si la pression tactile mesurée se situe à l'intérieur d'une plage prédéterminée d'une pression tactile,

en particulier, le dispositif de mesure de tension artérielle déterminant une pression de référence à l'intérieur de la plage prédéterminée de la pression tactile, et calculant un coefficient de correction pour la pression tactile mesurée sur la base de la pression de référence déterminée.

**9.** Appareil selon la revendication 1, comprenant en outre un capteur de température conçu pour mesurer une température du corps,
le dispositif de mesure de tension artérielle corrigeant une erreur de la tension artérielle mesurée sur la base de la température mesurée et d'une relation entre la température corporelle et la tension artérielle.

**10.** Appareil selon l'une quelconque des revendications 1 à 9,
le dispositif de mesure de tension artérielle calculant une vitesse d'onde d'impulsion sur la base de la différence de phase, et estimant la tension artérielle sur la base d'une relation entre la vitesse d'onde d'impulsion calculée et la tension artérielle.

**11.** Appareil de la revendication selon l'une quelconque des revendications 1 à 9, comprenant en outre un capteur de reconnaissance d'empreintes digitales conçu pour reconnaître les empreintes digitales,
le processeur étant en outre conçu pour mettre en marche sélectivement l'une ou plusieurs des sources de lumière sur la base d'au moins l'un d'une forme de contact, d'une zone de contact, et d'un motif d'empreintes digitales identifié par les empreintes digitales reconnues.

**12.** Appareil selon la revendication 11,

le processeur étant en outre conçu pour fournir à l'utilisateur des informations sur une position de contact appropriée dans laquelle un doigt de l'utilisateur doit être placé pour mesurer la tension artérielle, et/ou
le processeur étant en outre conçu pour mettre en marche une source de lumière qui est plus proche de la zone de contact que d'autres sources de lumière parmi la pluralité des sources de lumière, et/ou
le processeur comprenant un stockage conçu pour reconnaître des utilisateurs individuels sur la base des empreintes digitales reconnues, et stocker la tension artérielle mesurée pour les utilisateurs individuels, et/ou
le processeur étant en outre conçu pour déterminer une position prédéterminée d'un doigt comme position d'émission de lumière sur la base du motif d'empreintes digitales et mettre en marche les une ou plusieurs sources de lumière pour émettre les lumières sur la position d'émission de lumière parmi la pluralité des sources de lumière du réseau de sources de lumière,
en particulier, le processeur étant en outre conçu pour déterminer la position d'émission de lumière sur la base d'une localisation d'une source de lumière qui maximise la différence de phase.

**13.** Appareil selon la revendication 1,

les une ou plusieurs lumières comprenant une première lumière d'une première longueur d'onde et une seconde lumière d'une seconde longueur d'onde qui est plus courte que la première longueur d'onde ;
les signaux PPG comprenant un premier signal de photopléthysmographie (PPG) et un second signal PPG respectivement de la part de la première lumière reçue et de la seconde lumière reçue ; et
la différence de phase étant une différence de phase entre le premier signal PPG et le second signal PPG.

**14.** Dispositif de mesure de tension artérielle selon la revendication 13, les une ou plusieurs lumières comprenant en outre une troisième lumière d'une troisième longueur d'onde qui est plus courte que la première longueur d'onde et plus longue que la seconde longueur d'onde,

en particulier,
la première lumière étant émise par un premier émetteur de lumière, la seconde lumière étant émise par un second émetteur de lumière, et la troisième lumière étant émise par un troisième émetteur de lumière, et

le second émetteur de lumière, le troisième émetteur de lumière, et le premier émetteur de lumière étant respectivement positionnés dans un ordre de distance croissante depuis le détecteur de lumière, et/ou
le premier émetteur de lumière, le second émetteur de lumière, le troisième émetteur de lumière correspondant à une diode d'émission de lumière rouge (DEL), une DEL bleue, et une DEL verte, respectivement.

FIG. 1

# FIG. 2

# FIG. 3

# FIG. 4

BODY PART (e.g., FINGER)

PULSE WAVE
TRANSMISSION

FIRST LIGHT SOURCE

SECOND LIGHT SOURCE

LIGHT RECEIVER

# FIG. 5

BODY PART (e.g., FINGER)

PULSE WAVE
TRANSMISSION

$l$

FIRST LIGHT
SOURCE

THIRD LIGHT
SOURCE

SECOND LIGHT
SOURCE

LIGHT
RECEIVER

FIG. 6

# FIG. 7

Filter Layer (mosaic)

Sensor array

FIG. 8

FIG. 9

# FIG. 10

BODY PART (e.g., FINGER)

PULSE WAVE
TRANSMISSION

*l*

| LIGHT SOURCE | LIGHT RECEIVER |
|---|---|
| PRESSURE SENSOR | |

# FIG. 11

# FIG. 12

FIG. 13

## FIG. 14

## FIG. 15

BODY PART (e.g., FINGER)

PULSE WAVE
TRANSMISSION

FINGERPRINT
RECOGNITION
SENSOR

LIGHT
RECEIVER

FIRST
LIGHT
SOURCE

SECOND
LIGHT
SOURCE

LIGHT
SOURCE
ARRAY

BODY PART (e.g., FINGER)

PULSE WAVE
TRANSMISSION

LIGHT
RECEIVER

FIRST
LIGHT
SOURCE

SECOND
LIGHT
SOURCE

# FIG. 16

EP 3 153 094 B1

FIG. 17

31

**REFERENCES CITED IN THE DESCRIPTION**

**Patent documents cited in the description**

- US 20150057511 A1

- US 20050228296 A1